# EUROPEAN PATENT SPECIFICATION

(11) **EP 2 387 387 B1**
(45) Date of publication and mention of the grant of the patent: **11.11.2015**
(21) Application number: 10703428.2
(22) Date of filing: 15.01.2010
(51) Int. Cl.: A61K 8/02, A61K 8/81, A61K 8/58, A61Q 15/00, A61K 8/892

(54) **ANTIPERSPIRANT STICK COMPOSITION**
SCHWEISSHEMMENDE STICKZUSAMMENSETZUNG
COMPOSITION DE BÂTON ANTITRANSPIRATION

(30) Priority: 15.01.2009 EP 09150658
(43) Date of publication of application: 23.11.2011
(73) Proprietor: Coty Germany GmbH, 55116 Mainz (DE)
(72) Inventor: MATEU, Juan, R., Oak Ridge, New Jersey 07438 (US); BARONE, Salvatore, J., Staten Island, New York 10309 (US); MACCHIO, Ralph, Sparta, New Jersey 07871 (US)
(74) Representative: Gulde & Partner
(86) International application number: PCT/EP2010/050476
(87) International publication number: WO 2010/081886

(56) References cited:
- EP-A- 0 739 928
- EP-A- 0 848 039
- EP-A- 1 074 577
- WO-A-02/47639
- WO-A1-2006/028612
- WO-A1-2009/100974
- WO-A2-2008/074850
- US-A- 3 715 334
- US-B1- 6 238 656
- US-B1- 6 682 749

## Description

The present invention relates to an antiperspirant cosmetic composition for topical application to the skin comprising an effective amount of at least one antiperspirant active material, at least one gelling agent, at least one wax and at least one solvent for the at least one antiperspirant active material, wherein the at least one gelling agent is obtainable by the in-situ dehydrogenative coupling reaction of a siloxane polymer with at least two hydroxy-functionalized terminal groups or hydroxy-functionalized side chains and a polyol with at least two hydroxyl groups, a hydrosiloxane with at least two Si-H units and a catalyst.

Topical applied antiperspirant compositions are widely used all over the world in order to avoid or minimize visible wet patches on the skin, in particular in the axillary regions. Suitable antiperspirants are metal salts, such as salts of aluminum and zirconium. Usually these salts can also be used as deodorants avoiding the degradation of the perspiration by bacteria.

Antiperspirant formulations are provided in different physical forms, such as solids, gels, creams, lotions or sprays, but solids sticks are very popular by the consumers. There are three classes of solid antiperspirant sticks, namely emulsion sticks, suspension sticks and solution sticks. In the last two forms the antiperspirant is suspended or solved in a structured carrier, in particular in natural or synthetic waxes. The resulting sticks show a soft solid or a firm solid form.

However, wax structured compositions tend to leave visible white deposits when applied to human skin and the deposits can also be transferred onto clothing by physical contact with the skin. A significant proportion of consumers of antiperspirants have indicated displeasure at visible deposits. Thus, EP 0 942 707 and EP 1 267 821 provide low residue antiperspirants based on silicone elastomer material.

According to EP 0 942 707 a volatile silicone, a structurant and a cross-linked non-emulsifying siloxane elastomer are needed to form a stable antiperspirant stick composition. The cross-linked siloxane polymer is formed from the hydrosilation of vinyl silicone fluids by hydrosiloxane or MQ hydride fluids. Using further long chain water insoluble aliphatic alcohols as structurants a stable stick composition is achieved.

EP 1 267 821 further formulates polyethylene beads and an emollient into the stick composition to improve the stability of the composition and to impart a soothing and softening effect to the skin by the emollients. The resulting stick is a soft solid which leaves little or no white residue when applied.

EP 1 074 577 discloses silicone compositions that are functionalized with 1,2 or 1,3 glycol bearing substituents by hydrosilylation reaction. Antiperspirant salts are soluble in these silicone compositions so that transparent antiperspirant gel sticks can be produced.

EP 0 739 928 introduces a silicone latex consisting of a crosslinked polysiloxane dispersion comprising a siloxane polymer, polymer mixture or polymer/solvent mixture which are crosslinked by condensation, addition or free radical reactions. According to the method of EP 0 739 928 crosslinked emulsions with optimum handling characteristics can be produced where no thickeners are necessary for controlling rheology.

In US 6 238656 B1 a cosmetic raw material is described which is a silicone oil emulsion containing crosslinked silicone particles in silicone oil drops dispersed in water. This cosmetic raw material can be used in combination with other cosmetic raw materials resulting in cosmetic products which have good feeling on fingers and skin and good spreading characteristics.

It is the object of the present invention to provide an alternative solid and stable antiperspirant composition which leaves little residue when applied and imparts a good and soft feeling to the skin.

The present invention provides an antiperspirant cosmetic composition for topical application to the skin comprising an effective amount of at least one antiperspirant active material, at least one gelling agent, at least one wax and at least one solvent for the at least one antiperspirant active material, wherein the at least one gelling agent is obtainable by the in-situ dehydrogenative coupling reaction of a siloxane polymer with at least two hydroxy-functionalized terminal groups or hydroxy-functionalized side chains and a polyol with at least two hydroxyl groups, a hydrosiloxane with at least two Si-H units and a catalyst comprising a metal selected from the transition metals of the VIII B group of the chemistry's periodic table.

Using the in-situ dehydrogenative coupling reaction the antiperspirant cosmetic composition is solidified. During said in-situ reaction the gelling agent forms a gel matrix incorporating the other ingredients, such as the antiperspirant active material, the wax and/or the solvent. Thereby the antiperspirant active material, the wax and/or the solvent are well distributed and fixed at the same time. Thus, it is very important that the gelling reaction of the gelling agent, i.e. the solidification of the stick takes place in the composition after adding of all compounds. Advantageously, the antiperspirant composition of the present invention is provided as a solid stick which shows an improved stability compared to the sticks of the state of the art. In particular the long time stability of the antiperspirant stick is excellent. No segregation or bleeding of the active ingredients and/or the solvents are observed because the solvents are gelled within the gel matrix. Thus, the antiperspirant stick according to the invention leaves excellently low visible deposits on the skin and/or on the clothes.

The gel product of the in-situ dehydrogenative coupling reaction according to the invention is an organic/silicone gel which shows increased solubility, viscosity, wear and shine compared to the silicone compounds alone. During the in-situ reaction an additional structure inside the cosmetic composition is established that couples with the existing conventional wax systems. Thus, wax systems are enclosed inside the stick composition and cannot bleed out, so that waxes can also be formulated into the antiperspirant composition of the invention.

It was found by the inventors that the favorable properties of the present invention are only achieved by making the gelling agent in an in-situ reaction. During the dehydrogenative coupling reaction hydrogen is produced which escapes from the composition. Thereby the composition is further mixed so that the ingredients, in particular the antiperspirants, are well distributed. Surprisingly, during the gelling process the solvents are incorporated into the gel and become part of it. Thus, the gelling agent acts as a viscosity modifier and aids in suspending the antiperspirants. As a result the antiperspirant actives which are carried by the solvents are distributed homogeneously.

Due to the better distribution the antiperspirants are released homogeneously over a longer period of time. The antiperspirant activity of the compositions of the present invention is improved compared to the compositions of the state of the art.

It is a further advantage of the present invention that the aesthetics of the composition can also be manipulated beyond what is possible with the technologies of the state of the art. Due to the excellent stability of the composition additional ingredients to impart a better skin feeling or to improve the release of the antiperspirants can be formulated in the stick composition of the present invention. All additional ingredients become part of the gel matrix during the gelling procedure as well. Thus, the antiperspirants of the present invention are very effective and impart a smooth and soft feeling to the skin.

In a preferred embodiment of the invention a siloxane polymer with at least two hydroxy-functionalized terminal groups or hydroxy-functionalized side chains is used to form the gelling agent. Suitable siloxane polymers are for instance dimethiconol, a dimethiconol copolymer or a derivative thereof or mixtures thereof, in particular dimethiconol solution in cyclomethicone.

In another embodiment a hydroxy-functionalized silicate resin can be used as the siloxane polymer. The silicate resin is for example a silicone silicate copolymer, preferably a dimethiconol silicone silicate copolymer and most preferred trimethylsiloxysilicate.

A polyol with at least two hydroxyl groups is used as reactant for the dehydrogenative coupling reaction of the invention. The polyol is a copolymerized hydroxyl functionalized vegetable or synthetic oil or a mixture thereof. Suitable vegetable oils according to the invention are for instance olive oil, castor oil, sunflower oil, linseed oil, tall oil, soybean oil, colza oil, fish oil, cottonseed oil, palm oil, palm kernel oil, coconut oil, canola oil, flaxseed oil, high oleic canola oil, safflower oil or every other known saturated and unsaturated vegetable oil. Preferred synthetic oils are paraffin oils, such as e.g. iso-paraffin, ozokerite or ceresine or silicone oils, such as e.g. dimethicone or cyclomethicone.

Preferably the copolymerized hydroxyl functionalized vegetable or synthetic oils are copolymerised with an ester, a diester, a triester, urethanes or dimer diols, or mixtures thereof. Surprisingly, it was found that the copolymerized hydroxyl functionalized vegetable or synthetic oils can be used as a polyol according to the invention. If the hydroxyl functionalized vegetable or synthetic oils are used directly the reactants do not react properly and a weak paste is achieved. Only a slight increase of the viscosity is observed. In contrast, if the copolymerized hydroxyl functionalized vegetable or synthetic oils are reacted together with a hydrosiloxane a very strong gel is achieved. The resulting gels show a paste-solid/rubbery texture.

Every known vegetable or synthetic oil can be used to form the copolymers according to the invention. Suitably copolymers are for instance diisostearyl polyglyceryl-3-dimer dilinoleate (Schercemol™ PDD), triisostearyl polyglyceryl-3-dimer dilinoleate (Schercemol™ TPID), hydrogenated castor oil dimer dilinoleate (Risocast® DA-H or Risocast® DA-L), hydrogenated castor oil isopropyl dimer dilinoleate, hydrogenated castor oil/sebacic acid copolymer (Crodabond CSA), castor oil/3-isocyanateomethyl-3,5,5-trimethylcyclohexyl isocyanate copolymer (Castor Oil/IPDI Copolymer, Polyderm® PPI-CO), castor oil/dimer diol copolymer or castor oil/isocetyl alcohol copolymer, or a mixture thereof.

According to the invention the use of compounds with more than two hydroxyl groups is preferred. The higher number of reactive sites, i.e. the higher number of hydroxyl groups, increases the reaction rate of the gelling agent forming reaction and allows the reduction of the catalysts. Further the gel strength of the cross-linked gel is increased. Thus, the number of functional groups in the reactive compounds is varied in order to improve the solubility of the resulting gel.

In a preferred embodiment a copolymer of a hydroxyl functionalized vegetable or synthetic oil and isocetyl alcohol and/or dimer diols is used. These copolymers show an improved reaction. Directly after contact of the compounds the reaction starts so that no further mixing is needed.

The siloxane polymer and the copolymerized hydroxyl functionalized vegetable or synthetic oils, are used together to form the gelling agent. This is highly advantageous because the copolymerized hydroxyl functionalized vegetable or synthetic oils can be used directly as solvents for the antiperspirant, so that no further solvents, such as silicone oils are needed. That means all silicone compounds are incorporated into the gelling agent and after the dehydrogenative coupling reaction free silicone oil is not present in the antiperspirant stick any more. The resulting gel is a silicone/organic compound which shows a better compatibility with human skin than silicone compounds alone. Thus, these cosmetic compositions are also applicable to human skin which is very sensitive and/or shows allergic reactions to silicone compounds, including silicone solvents.

Thus, the siloxane polymer and the polyol, in particular the copolymerized hydroxyl functionalized vegetable or synthetic oils, are used as a mixture. A suitable ratio of the two components is from 1:5 to 5:1 siloxane polymer to polyol. Most preferred the siloxane polymer and the polyol are mixed in a ratio of 2:1 or in equal amounts, i.e. in a ratio of 1:1. Using a mixture of the organic polyols and the silicone polymers the compatibility parameters can be extended. To form the gelling agent according to the invention a hydrosiloxane and a catalyst are further needed. As a suitable hydrosiloxane polymethyl hydrosiloxane is preferably used, but other cosmetically acceptable hydrosiloxanes can be used according to the invention.

In a preferred embodiment the molecular weight of the reactants is used to determine the physical properties of the cosmetic composition according to the invention. A higher molecular weight increases the stability of the cosmetic stick. Furthermore a better skin feeling is obtained.

As a catalyst every catalyst is preferred which is able to catalyze a dehydrogenative coupling reaction. In particular, a metal selected from the transition metals of the VIII B group of the chemistry's periodic table is used. Preferably platinum or palladium are used. The most preferred catalyst according to the invention is platinum-divinyl tetramethyldisiloxane complex.

According to the invention the antiperspirant stick comprises 1 to 95 percent by weight, preferably 1 to 70 percent by weight, more preferred 1 to 50 percent by weight and most preferred 5 to 20 percent by weight of the gelling agent based on the total weight of the composition, whereby the term gelling agent means the product of the in-situ dehydrogenative coupling reaction.

According to the invention the gelling agent is formed in-situ so that the individual reactants have to be formulated into the cosmetic composition of the present invention. All weight percents given for the reactants are based on the total weight of the antiperspirant composition.

In a preferred embodiment of the invention the hydroxy functionalized siloxane polymer and/or polyol is contained in an amount of at about 0.5 to about 94 weight %, preferably at about 1 to about 50 weight %, more preferred at about 5 to about 25 weight % and most preferred about 10 weight %.

The hydrosiloxane is preferably contained in an amount of at about 0.5 to about 94 weight %, preferably at about 1 to about 30 weight %, more preferred at about 1 to about 15 weight % and most preferred about 5 weight %.

The catalyst is preferably contained in an amount of at about 0.001 to about 3 weight %, in particular at about 0.01 to about 2 weight %, more preferred at about 0.1 to about 1 weight % and most preferred about 0.5 weight %.

According to the invention the antiperspirant cosmetic composition comprises a suitable amount of an active antiperspirant. In a preferred embodiment the antiperspirant active material is an astringent, preferably an aluminum salt, a zirconium salt or an aluminum and/or zirconium complex, or a mixture thereof. According to the invention inorganic salts or salts with organic anions and complexes can be used as astringent compound.

Suitable astringent compounds include aluminum halides, such as e.g. aluminum chloride, zirconium halides, aluminum/zirconium halides and halohydrate salts, such as chlorohydrate, aluminum hydroxy halides, zirconyl hydroxyhalides, zirconyl oxyhalides, zirconium oxycloride, zirconium hydroxy chloride, aluminum sulfocarbolate, aluminum sulfate, zinc sulfate or zinc sulfocarbolate, or mixtures thereof.

Aluminium halohydrates are usually defined by the general formula Al₂(OH)ₓQ_{y} · wH₂O in which Q represents chlorine, bromine or iodine, x is variable from 2 to 5 and x+y=6 while wH₂O represents a variable amount of hydration. According to the invention for instance aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG or a mixture thereof are preferred.

Zirconium actives can usually be represented by the empirical general formula ZrO(OH)_{2n-nz} B_{z} · wH₂O in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂O. Preferably B represents chloride and the variable z lies in the range from 1.5 to 1.87. In practice, such zirconium salts are usually not employed by themselves, for instance as zirconium chlorohydrate but as a component of a combined aluminium and zirconium-based antiperspirant, such as aluminum-zirconium chlorohydrate and/or derivatives thereof.

Antiperspirant complexes based on the above-mentioned astringent aluminium and/or zirconium salts are preferably used according to the invention. The complex often employs a compound with a carboxylate group and advantageously this is an amino acid. Examples of suitable amino acids include for instance tryptophan, beta-phenylalanine, valine, methionine beta-alanine and glycine.

It is highly desirable to employ complexes of a combination of aluminium halohydrates and zirconium chlorohydrates together with amino acids such as for instance glycine. Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are for instance aluminum-zirconium tetrachlorohydrex GLY, aluminum zirconium octachlorohydrate, aluminum zirconium octachlorohydrex GLY, aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY, or aluminum zirconium trichlorohydrate GLY or mixtures thereof.

Preferred types of astringent compounds according to the invention are e.g. aluminum chlorohydrates, aluminum-zirconium chlorohydrates or aluminum-zirconium trichlorohydrex glycine or mixtures thereof available from Reheis under the trade names Rezal 36 GP Superultrafine and Reach AZP 908.

The antiperspirant active material is contained in the cosmetic composition of the invention in an amount of 1 to 40 percent by weight, preferably 1 to 30 percent by weight, most preferred 4 to 25 percent by weight based on the total weight of the antiperspirant cosmetic composition.

In another embodiment of the present invention the composition also comprises a water soluble calcium salt, such as calcium chloride. Calcium salts are thought to boost the antiperspirant efficacy.

According to the invention the antiperspirant composition further contains cosmetically acceptable waxes. The waxes used in the antiperspirant cosmetic composition of the invention are solid at room temperature and are intended to improve the structure of the composition. Suitable compounds may be hydrocarbon-based waxes or silicone waxes, in particular of plant, mineral, animal and/or synthetic origin, or mixtures thereof. They may have a melting point of greater than 40°C and better still greater than 45°C. According to the in-situ dehydrogenative coupling reaction the waxes are incorporated into the gel matrix of the gelling agent. Thus, unintentionally bleeding out is avoided and waxes can also be used which are suspected to impart allergic reactions. Further, the transfer of solvents to the clothes is avoided.

All waxes which are generally used in personal care/cosmetic industry can be used. Suitable natural waxes are for instance beeswax, preferably PEG8-Beeswax, carnauba wax, candelilla wax, bayberry wax, ouricoury wax, Japan wax, cork fibre wax, sugarcane wax, rice wax, montan wax, paraffin, microcrystalline wax or ceresin. Suitable synthetic waxes are for instance polyethylene, in particular polyethylene with a molecular weight in the range of 250 to 500, preferably in the range of 300 to 450, or ozokerite, castor wax, Fischer-Tropsch wax, mineral waxes or emulsifying waxes or alternatively fatty acid esters, for instance octacosanyl stearate or glycerides that are solid until the required temperature. The waxes are contained at about 1 to about 20 weight %, preferably at about 5 to about 15 weight %, most preferred at about 10 weight % based on the total weight of the composition.

According to the invention any cosmetically acceptable hydrocarbon or silicone can be used as a solvent for the antiperspirant active. As hydrocarbons alkanes or isoalkanes are preferred, in particular isoparaffins, for instance isododecane, isodecane and isohexadecane or isohexyl neopentanoate, or mixtures thereof. Isododecane is preferred according to the invention. As silicone dimethicone and derivatives thereof, such as e.g. dimethiconol, amodimethicone, cyclomethicones, phenyl trimethicone, dimethicone copolyol, stearyl dimethicone, cetyl dimethicone, or mixtures thereof are preferred. After the dehydrogenative coupling reaction the solvents are incorporated into the gel matrix and unintentionally bleeding out of the solvents is avoided. Thus, also solvents can be used which are suspected to impart allergic reactions. Further, the transfer of solvents to the clothes is avoided.

According to the invention it is especially preferred to use dimethiconol in cyclomethicone silicone oil, preferably DC 1501 (Dow Corning) or cyclomethicone. But also other cosmetically acceptable solvents like esters or vegetable oils may be applied. The solvents are contained at about 1 to about 60 weight %, preferably at about 10 to about 50 weight %, most preferred at about 30 weight % based on the total weight of the composition.

In another preferred embodiment the cosmetic composition further comprises cosmetically acceptable auxiliary substances. Suitable cosmetically acceptable auxiliary substances according to the invention are for instance odour absorbing substances, anti-microbial actives, skin lightening agents, hair thinning agents, depilatories, cooling actives, skin conditioning agents, anti-inflammatory actives, anti-irritants, colorants, powders, fillers, natural and synthetic oils, esters, emulsifiers, emollients, preservatives or fragrances, or mixtures thereof.

As odour absorbing substances any known material can be used. Suitable materials are for instance natural fibres, wood shavings, coke, lignite, activated carbon, odour-absorbing granules, natural zeolite or artificial zeolite, or mixtures thereof. In particular, it is preferred to use the odour absorbing substances as a fine powder.

Suitable anti-microbial actives according to the invention include for instance zinc oxide, zinc hydroxide, zinc carbonate, zinc phenolsulfonate, magnesium oxide, magnesium hydroxide, magnesium carbonate, lanthanum oxide, lanthanum hydroxide, lanthanum carbonate or sodium bicarbonate, or combinations thereof.

Skin lightening agents reduce the melanine synthesis in the skin. Suitable substances known in the state of the art can be formulated into the cosmetic composition of the present invention. Preferably tyramine, guailol from *Callitris intratropica,* a pharmacologically active base in a formulation having a pH of 8.5 to 10.5 or hydrogen peroxide, or a mixture thereof is used according to the invention.

Hair thinning agents and depilatories disintegrate the hair structure, in particular the keratin fibres. Suitable actives, such as e.g. thioglycolic acid or thioglycolate are known by the skilled person.

To impart a fresh and comfortable feeling to the skin cooling actives can be formulated into the antiperspirant composition of the present application. A cooling effect to the skin further reduces perspiration. Suitable cooling actives according to the invention are menthol and its derivatives, for instance menthyl lactate (Frescolat ML) or menthone glycerin acetal (Frescolat MGA).

In a preferred embodiment skin conditioning agents, such as e.g. vitamins, mineral salts, trace elements, plant extracts, animal extracts, proteins or enzymes, or mixtures thereof are formulated into the cosmetic composition of the present invention. Suitable anti-irritants or anti-inflammatory actives used in the present invention are for instance dexpanthenol, zinkoxid, plant extracts, such as chamomille extract, tea tree extract or witch hazel extract, or mixtures thereof.

As a colorant any cosmetically acceptable pigment, natural or synthetic organic or inorganic dye, or mixtures thereof are preferred. According to the invention pigments are for instance white or coloured, mineral or organic particles that are insoluble in the solvent and which are intended to colour and/or to opacify the composition. Examples for mineral pigments which can be used according to the invention are made for instance of titanium oxide, titanium dioxide, zirconium oxide or cerium oxide, and also zinc oxide, iron (II; III) oxide, chromium oxide or bismuth oxychloride. Further, ferric blue, manganese violet, copper powder and bronze powder can be used. As organic pigments the use of carbon black, and barium, strontium, calcium (D & C Red No. 7) and aluminum lakes is preferred. Suitable dyes are soluble in organic solvents.

In another preferred embodiment the antiperspirant stick comprises powders, and/or mineral or synthetic particles of any form. As mineral particles for instance mica, talc, silica, chalk, fullers earth, starch, gums and kaolin are preferred. Synthetic particles can be made of acrylic acid polymers, such as e.g. polymethylmetacrylate (PMMA), polyamide, polyethylene, polyurethane, or mixtures thereof. As fillers any cosmetically acceptable filler known to a person skilled in the art can be used according to the invention. Powders are contained at about 0.5 to about 15 weight %, preferably, at about 1 to about 10 weight % based on the total weight of the composition.

Natural and synthetic oils which are used in the personal care/cosmetic industry are suitable for the antiperspirant stick composition according to the invention. In particular, hydrogenated oils, for instance jojoba oil, avocado oil, spermaceti oil, borage oil, thistle oil, groundnut oil, St. John's wort oil, coconut oil, sweet almond oil, evening primrose oil, ricinus oil, sesame oil, sun flower oil or wheat germ oil, or mixtures thereof are preferred.

As esters any cosmetically acceptable ester can be comprised as an auxiliary substance. Esters which are known for their antiseptic properties and which are conventionally used in this respect in compositions intended to be administered to man or animals as pharmaceutical, nutritional and cosmetic compositions are preferred. The presence of these compounds ensures stability over time for the corresponding compositions. Esters may also be utilized as a carrier, a skin conditioning agent or an emollient. Illustrative esters are those formed from C₁-C₂₀-alkanols esterified with C₈-C₂₂-alkanoic acids. Examples include isopropyl myristate, lauryl myristate, isopropyl palmitate, diisopropyl sebacate or diisopropyl adipate. Most preferred is isopropyl palmitate. Amounts of the ester may range from about 0.5 to about 30%, preferably from 5 to 20%, optimally from about 8 to about 15% by weight based on the total weight of the composition.

As emulsifier every cosmetically acceptable emulsifier known to the person skilled in the art can be used according to the invention. Suitable emulsifiers according to the invention are for instance Glyceryl Stearate, PEG-100 Stearate, such as Arlacel® 165V or stearyl alcohol.

Suitable preservatives are for instance parabens, phenoxyethanol, caprylyl glycol, such as Lexgard O (Caprylyl Glycol), Lexgard GMCY (Glyceryl Caprylate) or any typical preservative used in the personal care/cosmetic industry.

In a preferred embodiment of the invention the antiperspirant stick further comprises fragrances. Every known substance used as a fragrance can be formulated in the cosmetic stick of the present invention. In particular scents are preferred which transfer the feeling of freshness and wellness, such as e.g. fruit flavours or light flower flavours, or mixtures thereof.

In a preferred embodiment of the invention the antiperspirant stick composition comprises the antiperspirant actives, the reactants for the in-situ reaction forming the gelling agent, waxes, solvents and optionally skin conditioning agent, natural or synthetic oils or fragrances, or mixtures thereof. According to the invention the reactants are the hydroxy-functionalized siloxane polymer and polyol, the hydrosiloxane and the catalyst. All components are contained within the amounts which are mentioned above.

In a preferred embodiment of the invention the antiperspirant stick composition comprises the antiperspirant actives in an amount of 1 to 95 weight %, the reactants for the in-situ reaction forming the gelling agent in an amount of 1 to 50 weight %, waxes in an amount of 0.1 to 30 weight %, solvents in an amount of 1 to 60 weight %, and optionally skin conditioning agent in an amount of 0.1 to 50 weight %, natural or synthetic oils in an amount of 1 to 50 weight %, fragrances in an amount of 0.1 to 7.5 weight % or mixtures thereof.

In a more preferred embodiment of the invention the antiperspirant stick composition comprises the antiperspirant actives in an amount of 20 to 30 weight %, the reactants in an amount of 15 to 20 weight %, waxes in an amount of 7.5 to 12.5 weight %, solvents in an amount of 25 to 35 weight %, and optionally skin conditioning agent in an amount of 10 to 15 weight %, natural or synthetic oils in an amount of 10 to 15 weight %, fragrances in an amount of 2 to 6 weight % or mixtures thereof.

According to the invention the reactants are the hydroxy-functionalized siloxane polymer preferably contained in an amount of 0.4 to 30 weight % and the polyol preferably contained in an amount of 0.3 to 15 weight %, the hydrosiloxane preferably contained in an amount of 0.2 to 5 weight % and the catalyst preferably contained in an amount of 0.1 to 5 weight %.

In a more preferred embodiment the hydroxy-functionalized siloxane polymer is contained in an amount of 5 to 15 weight %, and the polyol is contained in an amount of 1 to 10 weight %, the hydrosiloxane is contained in an amount of 0.2 to 2 weight % and the catalyst is contained in an amount of 0.1 to 2 weight %.

In a more preferred embodiment of the invention the antiperspirant stick composition comprises the antiperspirant actives in an amount of 25 weight %, the reactants for the in-situ reaction in an amount of 16.2 weight %, waxes in an amount of 10 weight %, solvents in an amount of 30 weight % and a skin conditioning agent in an amount of 13,8 weight %. The reactants are preferably used in the following amounts. The hydroxy-functionalized siloxane polymer is used in an amount of 10 weight %, the polyol is used in an amount of 5 weight %, the hydrosiloxane is used in an amount of 1 weight % and the catalyst is used in an amount of 0.2 weight %.

In the most preferred embodiment the hydroxy-functionalized siloxane polymer is dimethiconol and the polyol is a copolymerized hydroxyl functionalized vegetable oil, more preferred hydrogenated castor oil dimer dilinoleate. The preferred hydrosiloxane is polymethylhydrosilane. As catalyst Platinum-divinyl tetramethyldisiloxane is preferred. As antiperspirant active Aluminium Zirconium Tetrachlorohydrex gly is preferably used and the wax is preferably a polyethylene wax. As solvent a cosmetically acceptable silicone oil, preferably cyclomethicone is used in the most preferred embodiment of the invention.

To form the gelling agent of the stick composition the hydroxy-functionalized siloxane and/or the polyol is mixed with the catalyst, the waxes, the solvents and optionally with the skin conditioning agent, natural or synthetic oils or fragrances, or mixtures of the optional ingredients. Last the hydrosiloxane is added and the gelling agent is formed immediately in-situ inside the cosmetic composition.

It is a further object of the present invention to provide a method of production of the antiperspirant stick according to the invention. The preparation of the antiperspirant stick is the same as for conventional product forms. The antiperspirant stick is prepared in a per se known manner.

In a preferred embodiment of the invention the method of making the antiperspirant cosmetic composition comprises the following steps. Heating of the at least one wax component under continuous stirring to about the melting temperature and stirring until the at least one wax is completely molten. Solving the at least one antiperspirant active material in the at least one solvent and adding the solvent to the molten wax. Adding the hydroxy-functional siloxane polymer and the polyol to the mixture and stirring of the mixture until homogeneity. After a homogeneous mixture is achieved the mixture is cooled down to 50 °C. Adding the catalyst comprising a metal selected from the transition metals of the VIII B group of the chemistry's periodic table to the mixture under continuous stirring until homogeneity. And optionally the method comprises the steps of adding of cosmetically and/or pharmaceutically auxiliary substances and stirring until homogeneity. The final step of the method of production is adding the hydrosiloxane to the mixture in order to start the dehydrogenative coupling reaction.

In a preferred embodiment the at least one wax component is added into an appropriate flask and heated under continuous stirring to about the melting temperature and stirred until the waxes are completely molten.

Then the solvent comprising the antiperspirant actives and optionally the skin conditioning agents and the hydroxy-functional siloxan polymer and/or the polyol are added. Optionally these ingredients can be premixed before adding. After a homogeneous mixture is achieved the temperature can be reduced to 50 °C, but the mixture does not start to solidify again. The initial over-heating is needed to ensure that all components are completely molten and can be distributed equally, but the catalyst may not be heated over 50 °C in order to maintain the catalytic activity.

If the composition is cooled down to a temperature lower than 50 °C the catalyst is added. Thereby the mixture is stirred or mixed continuously. If cosmetically and/or pharmaceutically auxiliary substances should be included into the mixture they are added last. The mixture is mixed until homogeneity, but at least for 15 minutes. Finally the temperature of the mixture is further reduced, but the mixture should not start to solidify again.

Finally the hydrosiloxane is added and after an initial stirring the in-situ reaction starts. Stirring and/or mixing and heating are stopped and the gelling agent is formed by a dehydrogenative coupling reaction. The composition is transferred to the stick mold and/or directly to the cosmetic applicator. Due to the formation of the gelling agent and cooling down to room temperature the composition solidifies completely and forms the stable antiperspirant stick of the present invention.

It is another object of the present invention to provide a antiperspirant stick together with an appropriate applicator.

The product obtainable by the in-situ dehydrogenative coupling reaction of a siloxane polymer with at least two hydroxy-functionalized terminal groups or hydroxy-functionalized side chains and a polyol with at least two hydroxyl groups, a hydrosiloxane with at least two Si-H units and a catalyst comprising a metal selected from the transition metals of the VIII B group of the chemistry's periodic table can be used as a gelling agent in solid cosmetic compositions, in particular in decorative cosmetics, preferably in lipsticks, foundations or concealing sticks. If the product of the in-situ reaction of the present invention is used as gelling agent in products of decorative cosmetics the compositions are formulated without the antiperspirant actives.

It is disclosed a gelling agent being the product obtainable by the in-situ dehydrogenative coupling reaction of a siloxane polymer with at least two hydroxy-functionalized terminal groups or hydroxy-functionalized side chains and a polyol with at least two hydroxyl groups, a hydrosiloxane with at least two Si-H units and a catalyst.

It is furthermore disclosed a method for incorporating active ingredients stably into solid cosmetic compositions. The method comprises solving the active ingredients in an appropriate solvent, adding the ingredients of the cosmetic composition and adding to the mixture a gelling agent obtainable by the in-situ dehydrogenative coupling reaction of a siloxane polymer with at least two hydroxy-functionalized terminal groups or hydroxy-functionalized side chains and a polyol with at least two hydroxyl groups, a hydrosiloxane with at least two Si-H units and a catalyst. The resulting compositions show a good long time stability and a homogeneous distribution of the active ingredients.

The following examples are offered to illustrate the cosmetic antiperspirant stick of the present invention. They are not intended to be limiting in any respect.

### Example 1

An antiperspirant stick according to the invention is prepared comprising.

| | |
|---|---|
| 0.1-95 weight % | Antiperspirant active material(s) |
| 1-50 weight % | Gelling agent |
| 0.1-30 weight % | Wax(es) |
| 1-60 weight % | Solvent(s) |
| 0.1-50 weight % | Skin conditioning agent(s) |
| 0.1-10 weight % | Fragrance(s) |

The antiperspirant stick of Example 1 is prepared by heating the wax(es) to about the melting temperature under continuous stirring until the wax(es) are completely molten. In parallel the antiperspirant active(s) are solved in the appropriate solvent(s) and mixed with the skin conditioning agent(s) and the hydroxy-functional siloxan polymer and/or the polyol. The resulting mixture is added to the molten wax(es) under continuous stirring. After a homogeneous mixture is achieved the temperature is reduced to 50 °C. If the composition is cooled down the catalyst and fragrance(s) are added and the mixture is stirred for at least 15 minutes. Finally the temperature of the mixture is further reduced near to the solidification temperature and the hydrosiloxane is added. Mixing and heating are stopped and the composition is transferred to the stick mold.

### Example 2

An antiperspirant stick according to the invention is prepared as shown in Example 1. The stick composition comprises.

| | | |
|---|---|---|
| 25 | weight % | antiperspirant active material(s) |
| 16.2 | weight % | gelling agent |
| 10 | weight % | wax(es) |
| 30 | weight % | solvent(s) |
| 13.8 | weight % | skin conditioning agent(s) |
| 5 | weight % | Fragrance(s) |

### Example 3

An antiperspirant stick according to the invention is prepared which comprises.

| | | |
|---|---|---|
| 25 | weight % | Aluminium Zirconium Tetrachlorohydrex gly |
| 10 | weight % | Dimethiconol |
| 5 | weight % | Hydrogenated Castor Oil Dimer Dilinoleate |
| 1 | weight % | Polymethylhydrosilane |
| 0.2 | weight % | Platinum-divinyl tetramethyldisiloxane |
| 10 | weight % | Polyethylene waxes |
| 30 | weight % | Cyclomethicone |
| 5 | weight % | Stearyl Alcohol |
| 8.8 | weight % | Polydecene |
| 5 | weight % | Fragrance |

The antiperspirant stick of Example 3 is prepared by heating the polyethylene waxes, a mixture of Jeenate 3H and Jeenate 4H in ratio of 1:1 (Jeen Int. Corp.) to 78 °C under continuous stirring until the polyethylene is completely molten. In parallel the aluminium zirconium tetrachlorohydrex GLY (Rezal 36G, Reheis) is solved in the cyclomethicone (Dow Corning 245 Fluid, Dow Corning) and mixed with the stearyl alcohol (stearyl alcohol NF, Jeen Int. Corp.), the polydecene (PureSyn 1000, ExxonMobil/Synthetics) and the mixture of dimethiconol (DC 1501, Dow Corning) and hydrogenated castor oil dimer dilineolate (Risocast DA-H, Kokyu Alcohol Kogyo). The resulting mixture is added to the molten polyethylene under continuous stirring. After a homogeneous mixture is achieved the temperature is reduced to 50 °C. If the composition is cooled down the platinum-divinyl tetramethyldisiloxane (platinum divinyl complex, United Chemicals Technologies, Inc.) and the fragrances are added and the mixture is stirred for at least 15 minutes. Finally the temperature of the mixture is further reduced near to the solidification temperature and the polymethylhydrosilane (trade name polymethylhydrosiloxane, Gelest) is added. Mixing and heating are stopped after a short stirring and the composition is transferred to the stick mold.

### Example 4

An antiperspirant stick according to the invention is prepared which comprises.

| | | |
|---|---|---|
| 25 | weight % | Aluminium Zirconium Tetrachlorohydrex gly |
| 10 | weight % | Dimethiconol |
| 15 | weight % | Hydrogenated Castor Oil Dimer Dilinoleate |
| 1 | weight % | Polymethylhydrosilane |
| 0.2 | weight % | Platinum Catalyst |
| 20 | weight % | Polyethylene waxes |
| 5 | weight % | Stearyl Alcohol |
| 18.8 | weight % | Polydecene |
| 5 | weight % | Fragrance |

The antiperspirant stick of Example 4 is prepared by heating the polyethylene waxes, a mixture of Jeenate 3H and Jeenate 4H in ratio of 1:1 (Jeen Int. Corp.) to 78 °C under continuous stirring until the polyethylene is completely molten. In parallel the aluminium zirconium tetrachlorohydrex GLY (Rezal 36G, Reheis) is solved in the polydecene (PureSyn 1000, ExxonMobil/Synthetics) and mixed with the stearyl alcohol (stearyl alcohol NF, Jeen Int. Corp.) and the mixture of dimethiconol (DC 1501, Dow Corning) and hydrogenated castor oil dimer dilineolate (Risocast DA-H, Kokyu Alcohol Kogyo). The resulting mixture is added to the molten polyethylene under continuous stirring. After a homogeneous mixture is achieved the temperature is reduced to 50 °C. If the composition is cooled down the platinum-divinyl tetramethyldisiloxane (platinum divinyl complex, United Chemicals Technologies, Inc.) and the fragrances are added and the mixture is stirred for at least 15 minutes. Finally the temperature of the mixture is further reduced near to the solidification temperature and the polymethylhydrosilane (trade name polymethylhydrosiloxane, Gelest) is added. Mixing and heating are stopped and the composition is transferred to the stick mold.

### Example 5

An antiperspirant stick according to the invention is prepared as shown in Example 4 without adding fragrances. The antiperspirant comprises:

| | |
|---|---|
| 25 weight % | Aluminium Zirconium Tetrachlorohydrex gly |
| 10 weight % | Dimethiconol |
| 15 weight % | Hydrogenated Castor Oil Dimer Dilinoleate |
| 1 weight % | Polymethylhydrosilane |
| 0.2 weight % | Platinum Catalyst |
| 20 weight % | Polyethylene waxes |
| 10 weight % | Stearyl Alcohol |
| 18.8 weight % | Polydecene |

### Example 6

Storage stability of antiperspirant sticks is very important. To determine the storage stability of the antiperspirant stick according to the invention a stress test was performed. The cosmetic core stick was stored at room temperature, at 5°C, at 37° C, at 45° C and at 45° C with 50% of relative humidity, under daylight and UV radiation for 8 weeks (standard test for cosmetic industry). After 8 weeks the stick of the invention does not show any changes in texture, colour, odour or viscosity. Wax or silicone oil separation was not observed.

The antiperspirant composition was further applied to the skin. The skin feeling remains smooth and soft. White or coloured residues at the skin were not observed and no transfer to clothes was detectable.

## Claims

1. An antiperspirant cosmetic composition for topical application to the skin comprising
an effective amount of at least one antiperspirant active material, at least one gelling agent, at least one wax and at least one solvent for the at least one antiperspirant active material, wherein the at least one gelling agent is obtainable by the in-situ dehydrogenative coupling reaction of a mixture of a siloxane polymer with at least two hydroxy-functionalized terminal groups or hydroxy-functionalized side chains and a polyol with at least two hydroxyl groups which is a copolymerized hydroxyl functionalized vegetable or synthetic oil, a hydrosiloxane with at least two Si-H units and a catalyst comprising a metal selected from the transition metals of the VIII B group of the chemistry's periodic table.

2. The antiperspirant cosmetic composition of claim 1, wherein the cosmetic composition comprises cosmetically acceptable auxiliary substances, preferably odour absorbing substances, anti-microbial actives, skin lightening agents, hair thinning agents, depilatories, cooling actives, skin conditioning agents, anti-inflammatory actives, anti-irritants, colorants, powders, fillers, natural and synthetic oils, esters, emulsifiers, emollients, preservatives or fragrances, or mixtures thereof.

3. The antiperspirant cosmetic composition of claims 1 or 2, wherein the hydroxy-functionalized siloxane polymer is dimethiconol, a dimethiconol copolymer, a derivative thereof or a hydroxy-functional silicate resin, preferably a silicone silicate copolymer, or mixtures thereof.

4. The antiperspirant cosmetic composition of any one of claim 1 to 3, wherein the silicone silicate copolymer is a dimethiconol silicone silicate copolymer, preferably trimethylsiloxysilicate.

5. The antiperspirant cosmetic composition of claims 1 to 4, wherein the copolymerized hydroxyl functionalized vegetable or synthetic oil is copolymerized with an ester, a diester, a triester, urethanes or dimer diols, or a mixture thereof.

6. The antiperspirant cosmetic composition of claim 5, wherein the copolymerized hydroxyl functionalized vegetable or synthetic oil is diisostearoyl polyglyceryl-3-dimer dilinoleate, triisostearoyl polyglyceryl-3-dimer dilinoleate, hydrogenated castor oil dimer dilinoleate, hydrogenated castor oil isopropyl dimer dilinoleate, hydrogenated castor oil/sebacic acid copolymer, castor Oil/IPDI Copolymer, castor oil/dimer diol copolymer or castor oil/isocetyl alcohol copolymer, or a mixture thereof.

7. The antiperspirant cosmetic composition of any one of claim 1 to 6, wherein the hydrosiloxane is polymethyl hydrosiloxane.

8. The antiperspirant cosmetic composition of any one of claim 1 to 7, wherein the catalyst comprises platinum or palladium, most preferred platinum-divinyl tetramethyldisiloxane complex.

9. The antiperspirant cosmetic composition of any one of claim 1 to 8, wherein the at least one gelling agent is contained in an amount of 1 to 95 percent by weight, preferably 1 to 50 percent by weight, most preferred 5 to 20 percent by weight based on the total weight of the composition.

10. The antiperspirant cosmetic composition of any one of claim 1 to 9, wherein the at least one antiperspirant active material is an astringent, preferably an aluminum salt, a zirconium salt or an aluminum and/or zirconium complex, or a mixture thereof.

11. The antiperspirant cosmetic composition of claim 10, wherein the at least one antiperspirant active material is aluminum zirconium tetrachlorohydrate glycine, aluminum halides, aluminum hydroxy halides, zirconyl oxyhalides, zirconyl hydroxyhalides, zirconium hydroxy chloride or zirconium oxycloride, or a mixture thereof.

12. The antiperspirant cosmetic composition of any one of claim 1 to 11, wherein the at least one antiperspirant active material is contained in 1 to 40 percent by weight, preferably 1 to 30 percent by weight, most preferred 4 to 25 percent by weight based on the total weight of the composition.

13. A method of producing a antiperspirant cosmetic composition of any one of claims 1 to 12 comprising,
- heating the at least one wax component under continuous stirring to about the melting temperature and stirring until the at least one wax is completely molten,
- solving the at least one antiperspirant active material in the at least one solvent, and adding the solvent to the molten wax,
- adding the hydroxy-functional siloxane polymer and the polyol,
- stirring the mixture until homogeneity and cooling down to 50 °C,
- adding the catalyst comprising a metal selected from the transition metals of the VIII B group of the chemistry's periodic table under continuous stirring until homogeneity,
- optionally adding cosmetically and/or pharmaceutically auxiliary substances and stirring until homogeneity and
- adding the hydrosiloxane to start the dehydrogenative coupling reaction.

## Patentansprüche

1. Schweißverhütende kosmetische Zusammensetzung zur topischen Aufbringung auf die Haut, umfassend:
eine wirksame Menge wenigstens eines schweißverhütenden Wirkstoffs, wenigstens ein Geliermittel, wenigstens ein Wachs und wenigstens ein Lösungsmittel für den wenigstens einen schweißverhütenden Wirkstoff, wobei das wenigstens eine Geliermittel erhältlich ist durch die dehydrierende In-situ-Kupplungsreaktion eines Gemischs aus einem Siloxanpolymer mit wenigstens zwei hydroxyfunktionalisierten Endgruppen oder hydroxyfunktionalisierten Seitenketten und einem Polyol mit wenigstens zwei Hydroxygruppen, das ein copolymerisiertes hydroxyfunktionalisiertes pflanzliches oder synthetisches Öl ist, einem Hydrosiloxan mit wenigstens zwei Si-H-Einheiten und einem Katalysator, der ein aus den Übergangsmetallen der Gruppe VIII B des Periodensystems der Chemie ausgewähltes Metall umfasst.

2. Schweißverhütende kosmetische Zusammensetzung nach Anspruch 1, wobei die kosmetische Zusammensetzung kosmetisch akzeptable Hilfsstoffe, vorzugsweise geruchsabsorbierende Stoffe, antimikrobielle Wirkstoffe, Hautaufheller, Haarausdünnungsmittel, Haarentfernungsmittel, kühlende Wirkstoffe, Hautkonditionierungsmittel, entzündungshemmende Wirkstoffe, reizlindernde Stoffe, Farbstoffe, Puder, Füllstoffe, natürliche und synthetische Öle, Ester, Emulgatoren, Emollientien, Konservierungsmittel oder Duftstoffe oder Gemische derselben, umfasst.

3. Schweißverhütende kosmetische Zusammensetzung nach den Ansprüchen 1 oder 2, wobei es sich bei dem hydroxyfunktionalisierten Siloxanpolymer um Dimethiconol, ein Dimethiconol-Copolymer, ein Derivat davon oder ein hydroxyfunktionelles Silicatharz, vorzugsweise ein Silicon-Silicat-Copolymer, oder Gemische derselben handelt.

4. Schweißverhütende kosmetische Zusammensetzung nach einem der Ansprüche 1 bis 3, wobei das Silicon-Silicat-Copolymer ein Dimethiconol-Silicon-Silicat-Copolymer, vorzugsweise Trimethylsiloxysilicat, ist.

5. Schweißverhütende kosmetische Zusammensetzung nach den Ansprüchen 1 bis 4, wobei das copolymerisierte hydroxyfunktionalisierte pflanzliche oder synthetische Öl mit einem Ester, einem Diester, einem Triester, Urethanen oder Dimerdiolen oder einem Gemisch derselben copolymerisiert ist.

6. Schweißverhütende kosmetische Zusammensetzung nach Anspruch 5, wobei das copolymerisierte hydroxyfunktionalisierte pflanzliche oder synthetische Öl Diisostearoyl Polyglyceryl-3 Dimer Dilinoleate, Triisostearoyl Polyglyceryl-3 Dimer Dilinoleate, Hydrogenated Castor Oil Dimer Dilinoleate, Hydrogenated Castor Oil Isopropyl Dimer Dilinoleate, Hydrogenated Castor Oil/Sebacic Acid Copolymer, Castor Oil/IPDI Copolymer, Castor Oil/Dimer Diol Copolymer oder Castor Oil/Isocetyl Alcohol Copolymer oder ein Gemisch derselben ist.

7. Schweißverhütende kosmetische Zusammensetzung nach einem der Ansprüche 1 bis 6, wobei das Hydrosiloxan Polymethylhydrosiloxan ist.

8. Schweißverhütende kosmetische Zusammensetzung nach einem der Ansprüche 1 bis 7, wobei der Katalysator Platin oder Palladium, ganz besonders bevorzugt Platin-Di-vinyltetramethyldisiloxan-Komplex, umfasst.

9. Schweißverhütende kosmetische Zusammensetzung nach einem der Ansprüche 1 bis 8, wobei das wenigstens eine Geliermittel in einer Menge von 1 bis 95 Gewichtsprozent, vorzugsweise 1 bis 50 Gewichtsprozent, ganz besonders bevorzugt 5 bis 20 Gewichtsprozent, basierend auf dem Gesamtgewicht der Zusammensetzung, enthalten ist.

10. Schweißverhütende kosmetische Zusammensetzung nach einem der Ansprüche 1 bis 9, wobei der wenigstens eine schweißverhütende Wirkstoff ein Adstringens, vorzugsweise ein Aluminiumsalz, ein Zirconiumsalz oder ein Aluminium- und/oder Zirconiumkomplex oder ein Gemisch derselben, ist.

11. Schweißverhütende kosmetische Zusammensetzung nach Anspruch 10, wobei es sich bei dem wenigstens einen schweißverhütenden Wirkstoff um Aluminum Zirconium Tetrachlorohydrate Glycine, Aluminiumhalogenide, Aluminiumhydroxyhalogenide, Zirconyloxyhalogenide, Zirconylhydroxyhalogenide, Zirconiumhydroxychlorid oder Zirconiumoxyclorid oder ein Gemisch derselben handelt.

12. Schweißverhütende kosmetische Zusammensetzung nach einem der Ansprüche 1 bis 11, wobei der wenigstens eine schweißverhütende Wirkstoff in 1 bis 40 Gewichtsprozent, vorzugsweise 1 bis 30 Gewichtsprozent, ganz besonders bevorzugt 4 bis 25 Gewichtsprozent, basierend auf dem Gesamtgewicht der Zusammensetzung, enthalten ist.

13. Verfahren zur Herstellung einer schweißverhütenden kosmetischen Zusammensetzung nach einem der Ansprüche 1 bis 12, umfassend:
- Erwärmen des wenigstens einen Wachsbestandteils unter kontinuierlichem Rühren auf ungefähr die Schmelztemperatur und Rühren, bis das wenigstens eine Wachs vollständig geschmolzen ist,
- Lösen des wenigstens einen schweißverhütenden Wirkstoffs in dem wenigstens einen Lösungsmittel und Zugeben des Lösungsmittels zu dem geschmolzenen Wachs,
- Zugeben des hydroxyfunktionellen Siloxanpolymers und des Polyols,
- Rühren des Gemischs bis zur Homogenität und Abkühlen auf 50°C,
- Zugeben des Katalysators, der ein aus den Übergangsmetallen der Gruppe VIII B des Periodensystems der Chemie ausgewähltes Metall umfasst, unter kontinuierlichem Rühren bis zur Homogenität,
- optional Zugeben kosmetischer und/oder pharmazeutischer Hilfsstoffe und Rühren bis zur Homogenität und
- Zugeben des Hydrosiloxans, um die dehydrierende Kupplungsreaktion zu starten.

## Revendications

1. Une composition cosmétique antiperspirante pour l'application topique sur la peau, comprenant
une quantité efficace d'au moins un matériau actif antiperspirant, au moins un agent gélifiant, au moins une cire et au moins un solvant pour l'au moins un matériau actif antiperspirant, l'au moins un agent gélifiant pouvant être obtenu par la réaction de couplage déshydrogénante in situ d'un mélange d'un polymère siloxane avec au moins deux groupes terminaux à fonction hydroxyle ou chaînes latérales à fonction hydroxyle et un polyol avec au moins deux groupes hydroxyles, lequel est une huile végétale ou synthétique à fonction hydroxyle copolymérisée, un hydrosiloxane avec au moins deux unités Si-H et un catalyseur comprenant un métal sélectionné parmi les métaux de transition du groupe 8 du tableau périodique des éléments.

2. La composition cosmétique antiperspirante de la revendication 1, la composition cosmétique comprenant des substances auxiliaires acceptables du point de vue cosmétique, de préférence des substances absorbant les odeurs, des agents actifs anti-microbiens, des agents d'éclaircissement de la peau, des agents d'amincissement des cheveux, des produits dépilatoires, des agents actifs rafraîchissants, des agents de conditionnement de la peau, des agents actifs anti-inflammatoires, des anti-irritants, des colorants, des poudres, des produits de remplissage, des huiles naturelles et synthétiques, des esters, des émulsifiants, des émollients, des agents de conservation ou des parfums, ou des mélanges de ceux-ci.

3. La composition cosmétique antiperspirante des revendications 1 ou 2, le polymère siloxane à fonction hydroxyle étant du diméthiconol, un copolymère de diméthiconol, un dérivé de ceux-ci ou une résine de silicate à fonction hydroxyle, de préférence un copolymère de silicate de silicone, ou des mélanges de ceux-ci.

4. La composition cosmétique antiperspirante selon l'une quelconque des revendications 1 à 3, le copolymère de silicate de silicone étant un copolymère de silicate de silicone de diméthiconol, de préférence du triméthylsiloxysilicate.

5. La composition cosmétique antiperspirante des revendications 1 à 4, l'huile végétale ou synthétique à fonction hydroxyle copolymérisée étant copolymérisée avec un ester, un diester, un triester, des uréthanes ou des diols dimères, ou un mélange de ceux-ci.

6. La composition cosmétique antiperspirante de la revendication 5, l'huile végétale ou synthétique à fonction hydroxyle copolymérisée étant du dilinoléate de polyglycéryl-3-dimère de diisostéaryle, du dilinoléate de polyglycéryl-3-dimère de triisostéaryle, du dilinoléate dimère d'huile de castor hydrogénée, du dilinoléate dimère d'isopropyle d'huile de castor hydrogénée, un copolymère d'acide sébacique/huile de castor hydrogénée, un copolymère d'IPDI/huile de castor, un copolymère de diol dimère/huile de castor ou un copolymère d'alcool isocétylique/huile de castor, ou un mélange de ceux-ci.

7. La composition cosmétique antiperspirante selon l'une quelconque des revendications 1 à 6, l'hydrosiloxane étant de l'hydrosiloxane de polyméthyle.

8. La composition cosmétique antiperspirante selon l'une quelconque des revendications 1 à 7, le catalyseur comprenant du platine ou du palladium, de préférence un complexe de tétraméthyldisiloxane de platine-divinyle.

9. La composition cosmétique antiperspirante selon l'une quelconque des revendications 1 à 8, l'au moins un agent gélifiant étant contenu dans une quantité de 1 à 95 % massique, de préférence de 1 à 50 % massique, de préférence de 5 à 20 % massique par rapport au poids total de la composition.

10. La composition cosmétique antiperspirante selon l'une quelconque des revendications 1 à 9, l'au moins un matériau actif antiperspirant étant un astringent, de préférence un sel d'aluminium, un sel de zirconium ou un complexe d'aluminium et/ou de zirconium, ou un mélange de ceux-ci.

11. La composition cosmétique antiperspirante de la revendication 10, l'au moins un matériau actif antiperspirant étant de la glycine de tétrachloro-hydrate de zirconium d'aluminium, des halogénures d'aluminium, des hydroxyhalogénures d'aluminium, des oxyhalogénures de zirconyle, des hydroxyhalogénures de zirconyle, des hydroxychlorures de zirconium ou des oxychlorures de zirconium, ou un mélange de ceux-ci.

12. La composition cosmétique antiperspirante selon l'une quelconque des revendications 1 à 11, l'au moins un matériau actif antiperspirant étant contenu dans une quantité de 1 à 40 % massique, de préférence de 1 à 30 % massique, de préférence de 4 à 25 % massique par rapport au poids total de la composition.

13. Une méthode pour produire une composition cosmétique antiperspirante selon l'une quelconque des revendications 1 à 12, comprenant
- chauffer l'au moins un composant à la cire en remuant continuellement jusqu'à la température de fusion, environ, et remuer jusqu'à ce que l'au moins une cire soit complètement fondue,
- faire dissoudre l'au moins un matériau actif antiperspirant dans l'au moins un solvant, et ajouter le solvant à la cire fondue,
- ajouter le polymère de siloxane à fonction hydroxyle et le polyol,
- remuer le mélange jusqu'à homogénéité et faire refroidir jusqu'à 50 °C,
- ajouter le catalyseur comprenant un métal sélectionné parmi les métaux de transition du groupe 8 du tableau périodique des éléments en remuant continuellement jusqu'à homogénéité,
- ajouter optionnellement des substances auxiliaires acceptables du point de vue cosmétique et/ou pharmaceutique et remuer jusqu'à homogénéité et
- ajouter l'hydrosiloxane pour démarrer la réaction de couplage déshydrogénante.
